# EUROPEAN PATENT APPLICATION

(11) **EP 1 025 836 A1**
(43) Date of publication of application: **09.08.2000**
(21) Application number: 00102539.4
(22) Date of filing: 07.02.2000
(51) Int. Cl.: A61K 7/42, C07C 229/30

(54) **Cosmetic light screening composition**

(30) Priority: 08.02.1999 EP 99102456
(71) Applicant: F. HOFFMANN-LA ROCHE AG, 4070 Basel (CH)
(72) Inventor: Bringhen, Alain, 1244 Choulex (CH); Gonzenbach, Hans Ulrich, 1202 Genève (CH); Schwarzenbach, Rolf, 8405 Winterthur (CH); Sidrac, Dominique, 74160 Saint-Julien (en Genevois) (FR)
(74) Representative: Kellenberger, Marcus Dr.

(57) **Abstract**

The invention relates to cosmetic light screening compositions containing in a cosmetically acceptable carrier
a) a compound of the general formula I 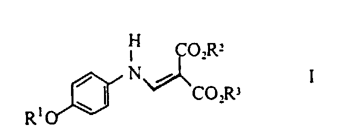 wherein
   - R¹: represents a C₁₋₈ straight or branched alkyl chain, and
   - R² and R³: each independently represent a C₁₋₈ straight or branched alkyl chain, and
   common UV-B screening agents and/or common UV-A screening agents.

## Description

The invention relates to cosmetic light screening compositions for the protection of skin and/or hair against ultraviolet radiation containing new synergistic mixtures of light screening compositions.

In particular the invention relates to cosmetic light screening compositions containing an anilinomethylene propanedioic acid ester and common UV-B screening and/or common UV-A screening agents.

It has now been found that a sunscreen composition containing in a cosmetically acceptable carrier
a) a compound of the general formula I 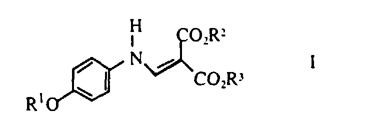 wherein
   - R¹: represents a C₁₋₈ straight or branched alkyl chain, and
   - R² and R³: each independently represent a C₁₋₈ straight or branched alkyl chain, and
b) common UV-B screening agents and/or common UV-A screening agents provides synergistically enhanced protection indices.

The term "C₁₋₈ straight or branched alkyl chain" refers to groups like methyl, ethyl, n-propyl, isopropyl, n-butyl, tert. butyl, pentyl, heptyl, 2-ethylhexyl and the like. A preferred branched C₋₈ alkyl chain is 2-ethylhexyl.

Preferred are compounds wherein R² and R³ each independently represent a straight C₁₋₄ alkyl chain, e.g. methyl, ethyl or n-butyl. Most preferred are compounds wherein R² and R³ are ethyl.

The group R¹ is preferably a straight C₁₋₄ alkyl chain, e.g. methyl, ethyl or n-butyl, more preferably ethyl.

The compounds of the general formula I and their manufacture are described in the European Patent Publication EP 0895776 A2.

The compounds of formula I have their absorption maxima between the classical UV-B and UV-A screening agents and especially and surprisingly increase the protective effect of common UV-B screening agents and/or common UV-A screening agents, although the absorption maxima of compounds of formula I do not lie in this region, but in the region of about 320 to 340 nm.

The term "common UV-B screening agents", i.e. substances having absorption maxima between about 290 and 320 nm, refers to the following UV-B screening agents:
--- Acrylates such as 2-ethylhexyl 2-cyano-3,3-diphenylacrylate (octocrylene, PARSOL 340), ethyl 2-cyano-3,3-diphenylacrylate and the like;
---Camphor derivatives such as 4-methyl benzylidene camphor (PARSOL 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
--- Cinnamate derivatives such as octyl methoxycinnamate (PARSOL MCX), ethoxyethyl methoxycinnamate, diethanolamine methoxycinnamate (PARSOL Hydro), isoamyl methoxycinnamate and the like as well as cinnamic acid derivatives bond to siloxanes;
---Organosiloxane compounds containing benzmalonate groups as described in the European Patent Publications EP 0358584 B1, EP 0538431 B1 and EP 0709080 A1;
--- Pigments such as microparticulated TiO₂, and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.
--- Imidazole derivatives such as e.g. 2-phenyl benzimidazole sulfonic acid and its salts (PARSOL HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like.
--- Salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like;
---Triazone derivatives such as octyl triazone (UVINUL T-150), dioctyl butamido triazone (UVASORB HEB) and the like.

The term "common UV-A screening agents", i.e. substances having absorption maxima between about 320 and 400 nm, refers to the following UV-A screening agents.
----Dibenzoylmethane derivatives such as 4-tert. butyl-4'-methoxydibenzoylmethane (PARSOL 1789), dimethoxydibenzoylmethane, isopropyldibenzoylmethane and the like;
----Benzotriazole derivatives such as 2,2'-methylene-bis-(6-(2H-benzotriazole-2-yl)-4-(1,1,3,3,-tetramethylbutyl)-phenol (TINOSORB M) and the like;
---Pigments such as microparticulated ZnO and the like. The term "microparticulated" refers to a particle size from about 5 nm to about 200 nm, particularly from about 15 nm to about 100 nm. The ZnO particles may also be coated by metal oxides such as e.g. aluminum or zirconium oxides or by organic coatings such as e.g. polyols, methicone, aluminum stearate, alkyl silane. Such coatings are well known in the art.

As dibenzoylmethane derivatives are photolabile it is necessary to photostabilze these UV-A screening agents. Thus, the term "common UV-A screening agent" also refers to dibenzoylmethane derivatives such as e.g. PARSOL 1789 stabilized by the following stabilizing agents:
--- 3,3-Diphenylacrylate derivatives as described in the European Patent Publications EP 0514491 B1 and EP 0780119 A1;
---- Benzyliden camphor derivatives as described in the US Patent Publication US 5 605 680;
---- Organosiloxanes containing benzmalonate groups as described in the European Patent Publications EP 0358584 B1, EP 0538431 B1 and EP 0709080 A1.

With regard to light screening compositions containing a compound of the general formula I and a common UV-B screening agent the following compositions are preferred:
a) a sunscreen composition containing in a cosmetically acceptable carrier a compound of the general formula I wherein R¹, R² and R³ are ethyl and octyl methoxy-cinnamate (PARSOL MCX);
b) a sunscreen composition containing in a cosmetically acceptable carrier a compound of the general formula I wherein R¹, R² and R³ are ethyl and the following polysiloxane compound of the general formula II described in the European Patent Publication EP 0709080 A1 and hereinafter referred to as "Polysiloxane A" 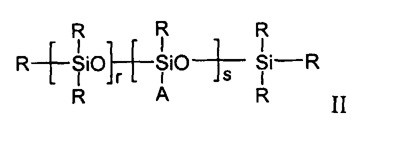 wherein
   - R: signifies methyl;
   - A: signifies a group of the formula IIa or IIb; 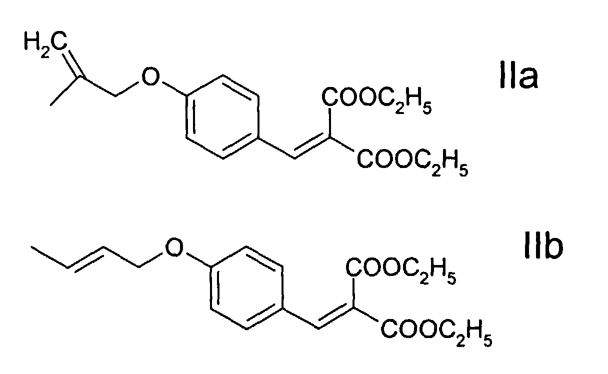
   - r: is a statistical mean value of about 4;
   - s: is a statistical mean value of about 60.
c) a sunscreen composition containing in a cosmetically acceptable carrier a compound of the general formula I wherein R¹, R² and R³ are ethyl and microparticulated TiO₂;
d) a sunscreen composition containing in a cosmetically acceptable carrier a compound of the general formula I wherein R¹, R² and R³ are ethyl and 4-methyl benzylidene camphor (PARSOL 5000);
e) a sunscreen composition containing a compound of the formula I wherein R¹, R² and R³ are ethyl and 2-ethylhexyl 2-cyano-3,3-diphenylacrylate (octocrylene, PARSOL 340).

With regard to light screening compositions containing a compound of the general formula I and a common UV-A screening agent the following compositions are preferred:
a) a sunscreen composition containing a compound of the formula I wherein R¹, R² and R³ are ethyl and 4-tert. butyl-4 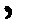 -methoxydibenzoyl-methane (PARSOL 1789) optionally stabilized by octocrylene (PARSOL 340), methylbenzylidene camphor (PARSOL 5000) or "Polysiloxane A";
b) a sunscreen composition containing a compound of the formula I wherein R¹, R² and R³ are ethyl and 2,2'-methylene-bis-(6-(2H-benzotriazole-2-yl)-4-(1,1,3,3,-tetramethylbutyl)-phenol (TINOSORB M);
c) a sunscreen composition containing a compound of the formula I wherein R¹, R² and R³ are ethyl and microparticulated ZnO.

The preparation of novel light screening compositions, especially of preparations for skin protection and, respectively, sunscreen preparations for everyday cosmetics is well known to the skilled artisan in this field and comprises incorporating a compound of formula I and common UV-B screening agents and/or common UV-A screening agents optionally together with stabilizers as described above in a cosmetic base which is usual for light screening agents.

The amount of compounds of the general formula I and common UV-B screening agents and/or common UV-A screening agents is not critical. Suitable amounts of each compound of the compositions described above are:
- Compound of the general formula I:: about 0.5 to about 10wt%,
- "Polysiloxane A":: about 0.5 to about 15wt%,
- PARSOL MCX:: about 0.5 to about 10wt%,
- PARSOL 340:: about 0.5 to about 10wt%,
- PARSOL 1789:: about 0.5 to about 5wt%,
- PARSOL 5000:: about 0.5 to about 4wt%,
- TINOSORB M:: about 0.5 to about 10wt%,
- TiO₂:: about 0.5 to about 25wt%,
- ZnO:: about 0.5 to about 20wt%.

Where convenient the light screening compositions of the present invention can also be combined with other conventional UV-A, and respectively, UV-B screening agents during this incorporation.

Thus, light screening compositions containing a compound of the general formula I and a common UV-B screening agent can be further combined with another common UV-A screening agent such as PARSOL 1789, ZnO, TINOSORB M and the like.

Furthermore, a light screening compositions containing a compound of the general formula I and a common UV-A screening agent can be further combined with another common UV-B screening agent such as PARSOL MCX, TiO₂, "Polysiloxane A" and the like.

The following Examples explain the invention in more detail but do not limit its scope in any manner.

In these examples the abbreviations and trade names selected have the following significance:
- ARLACEL 481: Glycerol sorbitan fatty acid ester sold by ICI
- ARLACEL P135: PEG-30 dipolyhydroxystearate sold by ICI
- ARLAMOL E: POP-(15)-stearyl alcohol sold by ICI
- ARLAMOL HD: Heptamethylnonane sold by ICI
- BHT: Butylhydroxytoluol (2,6 di-tert butyl-4-methyl phenol)
- BRIJ 72: POE-(2)-stearyl alcohol sold by ICI
- BRIJ 721: POE-(21)-stearyl alcohol sold by ICI
- CETIOL LC: Coco caprilate/caprate sold by Henkel
- Cetyl alcohol: Cetyl alcohol sold by Henkel
- CUTINA HR: Hydrogenated Castor oil sold by Henkel
- EDETA BD: Disodium EDTA sold by BASF
- ELFACOS C26: Hydroxyoctacosanyl hydroxystearate sold by Akzo Nobel
- FINSOL TN: C12-15 alkyl benzoate sold by Finetex Inc.
- KELTROL: Xanthan gum sold by Kelco
- KOH: Potassium hydroxyde sold by Merck
- LANETTE O: Cetearyl alcohol sold by Henkel
- NIPAGIN M: Methylparabene sold by Henkel sold by Nipa Ltd.
- PARSOL 340: Octocrylene sold by Roche
- PARSOL 1789: Butyl Methoxydibenzoylmethane sold by Roche
- PARSOL 5000: 4-Methyl Benzylidene Camphor sold by Roche
- PARSOL HS: 2-Phenyl benzimidazole sulfonic acid
- PARSOL MCX: Octyl Methoxycinnamate sold by Roche
- PEG 2 Stearate: Diethylene glycol monostearate sold by Croda
- PEMULEN TR-1: Acrylate/C10-30 alkyl acrylate crosspolymer sold byGoodrich
- Petroleum jelly: Petrolatum sold by Witco Corp.
- PHENONIP: mixture of 4-hydroxybenzoic acid esters sold by Nipa
- Propylene glycol: 1,2 propanediol sold by BASF
- RICINOL: Castor oil sold by Givaudan-Roure
- SATOL: Oleyl alcohol sold by Givaudan-Roure
- SILBIONE oil 70047 V20: Cyclomethicone sold by Rhône Poulenc
- SILICONE 1401: Cyclomethicone and dimethiconol sold by Dow Corning
- SILICONE 200/100 cs: Dimethicone sold by Dow Corning

### Example 1

### Preparation of 2-(4-Ethoxy-anilinomethylene)-propanedioic acid diethyl ester

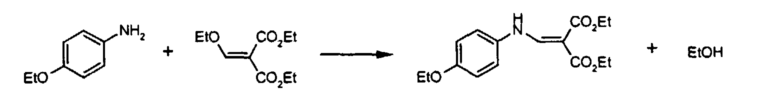

To 41.15g (0.3 Mol) of p-phenetidine in 90 ml of hexane, 64.9 g (0.3 Mol) of diethyl ethoxymethylenemalonate was added dropwise under stirring at room temperature. After an additional hour of stirring at room temperature, the reaction mixture was cooled with an ice bath and the product crystallized spontaneously. The crude solid material was isolated by filtration and washed with hexane to give 78.3 g of the title product; white solid melting at 55-56 °C, UV 329 nm (E=802).

### Example 2

### Preparation of a sunscreen O/W lotion containing 5% of UV-filter as described in Examples 3 to 8:

| Ingredients | % w/w |
|---|---|
| ARLAMOL E | 5.00 |
| ARLAMOL HD | 5.00 |
| BRIJ 72 | 3.00 |
| BRIJ 721 | 2.00 |
| UV-filter | 5.00 |
| ARLACEL P135 | 0.50 |
| LANETTE O | 5.00 |
| Stearic Acid | 1.50 |
| SILBIONE Oil 70047V20 | 1.00 |
| BHT | 0.10 |
| PHENONIP | 0.60 |
| Deionized Water qsp to | 100.00 |
| Xanthan Gum 1% sol'n | 6.00 |
| Propylene Glycol | 4.00 |
| UMORDAN P | 1.00 |

The organic phase containing the UV-filters was heated to 75°C, then the pre-heated aqueous phase (75° C) was added while stirring. The resulting emulsion was cooled to ambient temperature.

The following Examples 3 to 5 refer to sunscreen compositions according to the present invention containing a compound of the general formula I and a common UV-B screening agent.

### Example 3

Three sunscreen formulations according to Example 2 with the following UV-screening agents were prepared:
- Formulation n°1:: the UV-filter is the compound of Example 1 (2-(4-Ethoxy anilinomethylene)-propanedioic acid diethyl ester) at 5% w/w.
- Formulation n°2:: the UV-filter is PARSOL MCX (Octyl Methoxycinnamate) at 5%w/w.
- Formulation n°3:: the UV-filter is a mixture of 2.5 % w/w of the compound of Example 1 and 2.5 % w/w of PARSOL MCX.

### Determination of the sun protection factor:

For each formulation sun protecting factor (SPF) was determined according to the method described by B. Diffey and J. Robson in J. Soc. Cosmet. Chem. 40, 127-133 (1989).

The results are given in the following table:

| Sunscreen formulations | UV-filter | In-vitro SPF | Expected SPF | Synergy |
|---|---|---|---|---|
| 3/1 | 5% compound of Ex. 1 | 15.2 | | |
| 3/2 | 5% PARSOL MCX | 11.3 | | |
| 3/3 | 2.5% compound of Ex. 1 +2.5% PARSOL MCX | 19.8 | 13.3 | 49% |
| In vitro SPF: Mean value of 5 determinations. Expected SPF: Calculated by adding in vitro SPF-value of sunscreen formulation 1 and 2, divided by 2 (2.5% of each instead of 5%). Synergy: % deviation of in vitro SPF from expected (calculated) SPF. | | | | |

The combination of the compound of Example 1 and PARSOL MCX according to the invention shows an unproportional increase of the SPF. Usually, the SPF of filter combinations is very close to the calculated value from the performance of the single filters, therefore, we have a synergistic effect in this combination.

### Example 4

Three sunscreen formulations were prepared according to Example 2.
- Formulation n°1:: the UV-filter is the compound of Example 1 (2-(4-Ethoxy-anilinomethylene)-propanedioic acid diethyl ester) at 5% w/w.
- Formulation n°2:: the UV-filter is TiO₂ at 5% w/w.
- Formulation n°3:: the UV-filter is a mixture of 2.5 % w/w of the compound of Example 1 and 2.5 % w/w of TiO₂.

The results of the determination of the sun protection factor are given in the following table:

| Sunscreen formulations | UV-filter | In-vitro SPF | Expected SPF | Synergy |
|---|---|---|---|---|
| 4/1 | 5% compound of Ex. 1 | 15.2 | | |
| 4/2 | 5% TiO₂ | 1.3 | | |
| 4/3 | 2.5% compound of Ex.1 +2.5% TiO₂ | 16.0 | 8.3 | 94% |
| In vitro SPF: Mean value of 5 determinations. Expected SPF: Calculated by adding in vitro SPF-value of sunscreen formulation 1 and 2, divided by 2 (2.5% of each instead of 5%). Synergy: % deviation of in vitro SPF from expected (calculated) SPF. | | | | |

The combination of the compound of Example 1 and TiO₂ according to the invention shows an unproportional increase of the SPF. Usually, the SPF of filter combinations is very close to the calculated value from the performance of the single filters, therefore, we have a synergistic effect in this combination.

### Example 5

Three sunscreen formulations were prepared according to Example 2.
- Formulation n°1:: the UV-filter is the compound of Example 1 (2-(4-Ethoxy-anilinomethylene)-propanedioic acid diethyl ester) at 5% w/w.
- Formulation n°2:: the UV-filter is "Polysiloxane A" at 5% w/w.
- Formulation n°3:: the UV-filter is a mixture of 2.5 % w/w of the compound of Example 1 and 2.5 % w/w of "Polysiloxane A".

The results of the determination of the sun protection factor are given in the following table:

| Sunscreen formulations | UV-filter | In-vitro SPF | Expected SPF | Synergy |
|---|---|---|---|---|
| 5/1 | 5% compound of Ex.1 | 15.2 | | |
| 5/2 | 5% "Polysiloxane A" | 3.4 | | |
| 5/3 | 2.5% compound of Ex.1 +2.5% "Polysiloxane A". | 12.3 | 9.3 | 32% |
| In vitro SPF: Mean value of 5 determinations. Expected SPF: Calculated by adding in vitro SPF-value of sunscreen formulation 1 and 2, divided by 2 (2.5% of each instead of 5%). Synergy: % deviation of in vitro SPF from expected (calculated) SPF. | | | | |

The combination of the compound from Example 1 and "Polysiloxane A" shows an unproportional increase of the SPF. Usually, the SPF of filter combinations is very close to the calculated value from the performance of the single filters, therefore, we have a synergistic effect in this combination.

The following Examples 6 to 8 refer to sunscreen compositions according to the present invention containing a compound of the general formula I and a common UV-A screening agent. In Examples 7 and 8 PARSOL 1789 is stabilized.

### Example 6

Three sunscreen formulations were prepared according to Example 2.
- Formulation n°1:: the UV-filter is the compound of Example 1 (2-(4-Ethoxy-anilinomethylene)-propanedioic acid diethyl ester) at 5% w/w.
- Formulation n°2:: the UV-filter is PARSOL 1789 (Butyl Methoxydibenzoylmethane) at 2.5% w/w.
- Formulation n°3:: the UV-filter is a mixture of 2.5 % w/w of the compound of Example 1 and 1.25 % w/w of PARSOL 1789.

### Determination of the Erythemal UV-APF:

For each formulation Erythemal UV-APF; an indicator of UV-A protection property of a sunscreen product, calculated from MPF_{λ}, E_{λ}, B_{λ}. wherein
MPF_{λ}, = Monochromatic protection factor,
E_{λ} = Spectral irradiance of terrestrial sunlight under defined conditions (midday midsummer sunlight at 40°N, solar zenith angle 20°),
B_{λ} = Erythemal Effectiveness (CIE),
was determined according to the method described by B. Diffey and J. Robson in J. Soc. Cosmet. Chem. 40, 127-133 (1989).

The results of the determination of the Erythemal UV-A protection factor are given in the following table:

| Sunscreen formulations | UV-filter | In-vitro UV-APF | Expected UV-APF | Synergy |
|---|---|---|---|---|
| 6/1 | 5% compound of Ex.1 | 11.1 | | |
| 6/2 | 2.5% PARSOL 1789 | 9.7 | | |
| 6/3 | 2.5% compound of Ex. 1 +1.25% PARSOL 1789 | 24.0 | 10.4 | 131% |
| In vitro UV-A PF: Mean value of 5 determinations. Expected UV-A PF: Calculated by adding in vitro UV-APF-value of sunscreen formulation 1 and 2, divided by 2. Synergy: % deviation of in vitro UV-APF from expected (calculated) UV-A PF. | | | | |

The combination of the compound of Example 1 and PARSOL 1789 according to the invention shows an unproportional increase of the UV-APF. Usually, the UV-APF of filter combinations is very close to the calculated value from the performance of the single filters, therefore, we have a synergistic effect in this combination.

### Example 7

Three sunscreen formulations were prepared according to Example 2.
- Formulation n° 1:: the UV-filter is the compound of Example 1 (2-(4-Ethoxy-anilinomethylene)-propanedioic acid diethyl ester) at 5% w/w.
- Formulation n°2:: the UV-filter is a mixture of 1.7% w/w of PARSOL 1789 and 1.7%w/w of PARSOL 340.
- Formulation n°3:: the UV-filter is a mixture of 3 % w/w of the compound of Example 1 and 1 % w/w of PARSOL 1789 and 1% w/wof PARSOL 340.

The results of the determination of the sun protection factor and the Erythemal UV-A protection factor are given in the two following tables:

| Sunscreen formulations | UV-filter | In-vitro SPF | Expected SPF | Synergy |
|---|---|---|---|---|
| 7/1 | 5% compound of Ex.1 | 15.2 | | |
| 7/2 | 1.7% PARSOL 1789 + 1.7% PARSOL 340 | 3.4 | | |
| 7/3 | 3% compound of Ex.1, 1.7% PARSOL 1789 + 1.7% PARSOL 340 | 13.0 | 10.9 | 19% |
| In vitro SPF: Mean value of 5 determinations. Expected SPF: Calculated by adding in vitro SPF-value of sunscreen formulation 1 and 2, divided by 1.7. Synergy: % deviation of in vitro SPF from expected (calculated) SPF. | | | | |

| Sunscreen formulations | UV-filter | In-vitro UV-APF | Expected UV-APF | Synergy |
|---|---|---|---|---|
| 7/1 | 5% compound of Ex.1 | 11.1 | | |
| 7/2 | 1.7% PARSOL 1789 + 1.7% PARSOL 340 | 6.2 | | |
| 7/3 | 3% compound of Ex.1, 1.7% PARSOL 1789 + 1.7% PARSOL 340 | 11.5 | 10.2 | 13% |
| In vitro UV-APF: Mean value of 5 determinations. Expected UV-APF: Calculated by adding in vitro UV-APF -value of sunscreen formulation 1 and 2, divided by 1.7. Synergy: % deviation of in vitro UV-APF from expected (calculated) UV-APF. | | | | |

The combination of the compound of Example 1 and a mixture of PARSOL 1789 and PARSOL 340 according to the invention shows an unproportional increase of the SPF and of the UV-A PF as well. We have at the same time a synergistic effect of the UV-B and of the UV-A protection.

### Example 8

Three sunscreen formulations were prepared according to Example 2.
- Formulation n°1:: the UV-filter is the compound of Example 1 (2-(4-Ethoxy-anilinomethylene)-propanedioic acid diethyl ester) at 5% w/w.
- Formulation n°2:: the UV-filter is a mixture of 1.7% w/w of PARSOL 1789 and 1.7%w/w of PARSOL 5000.
- Formulation n°3:: the UV-filter is a mixture of 3 % w/w of the compound of Example 1 and 1 % w/w of PARSOL 1789 and 1%w/w of PARSOL 5000.

The results of the determination of the sun protection factor and the Erythemal UV-A protection factor are given in the two following tables:

| Sunscreen formulations | UV-filter | In-vitro SPF | Expected SPF | Synergy |
|---|---|---|---|---|
| 8/1 | 5% compound of Ex.1 | 15.2 | | |
| 8/2 | 1.7% PARSOL 1789 + 1.7% PARSOL 5000 | 7.9 | | |
| 8/3 | 3% compound of Ex.1, 1.7% PARSOL 1789 + 1.7% PARSOL 5000 | 17.2 | 13.6 | 27% |
| In vitro SPF: Mean value of 5 determinations. Expected SPF: Calculated by adding in vitro SPF-value of sunscreen Formulation 1 and 2, divided by 1.7. Synergy: % deviation of in vitro SPF from expected (calculated) SPF. | | | | |

| Sunscreen formulations | UV-filter | In-vitro UV-A PF | Expected UV-A PF | Synergy |
|---|---|---|---|---|
| 8/1 | 5% compound of Ex.1 | 11.1 | | |
| 8/2 | 1.7% PARSOL 1789 + 1.7% PARSOL 5000 | 6.4 | | |
| 8/3 | 3% compound of Ex.1, 1.7% PARSOL 1789 + 1.7% PARSOL 5000 | 12.5 | 10.3 | 21% |
| In vitro UV-A PF: Mean value of 5 determinations. Expected UV-A PF: Calculated by adding in vitro UV-A PF -value of sunscreen Formulation 1 and 2, divided by 1.7. Synergy: % deviation of in vitro UV-APF from expected (calculated) UV-APF. | | | | |

The combination of the compound of Example 1 and a mixture of PARSOL 1789 and PARSOL 5000 according to the invention shows an unproportional increase of the SPF and of the UV-A PF as well. We have at the same time a synergistic effect of the UV-B and of the UV-A protection.

Examples 9 to 12 refer to different sunscreen formulations according to the present invention.

### Example 9

A sunscreen oil in water lotion was prepared with the following ingredient

| Part | Ingredients | % w/w |
|---|---|---|
| A) | BRIJ 72 | 3.00 |
| | BRIJ 721 | 2.00 |
| | PEG 2 Stearate | 0.25 |
| | Compound of Example 1 | 2.50 |
| | PARSOL MCX | 7.50 |
| | Cetyl Alcohol | 1.00 |
| | CETIOL LC | 7.00 |
| | EDETA BD | 0.10 |
| | PHENONIP | 0.60 |
| | | |
| B) | Deionized Water | qsp 100 |
| | PEMULEN TR-1 (1% sol'n) | 20.00 |
| | Propylene Glycol | 5.00 |
| | NIPAGIN M | 0.15 |
| | KOH (10% sol'n) | qsp pH7 |

The organic phase A) containing the UV-filters was heated to 75°C, then the preheated aqueous phase B) (75°C) was added while stirring. The resulting emulsion was cooled to ambient temperature. The pH value was corrected with KOH 10%

### Example 10

A sunscreen water in SILICONE emulsion was prepared with the following ingredients:

| Part | Ingredients | %w/w |
|---|---|---|
| A) | SILICONE 1401 Substantivity Aid Fluid | 10.00 |
| | SILICONE 3225C Formulation Aid | 10.00 |
| B) | Compound of Example 1 | 5.00 |
| | TiO₂ | 5.00 |
| | ZnO | 2.00 |
| | FINSOLv TN | 5.00 |
| | EDETA BD | 0.10 |
| | PHENONIP | 0.60 |
| | | |
| C) | Deionized Water | Qsp 100 |
| | Propylene Glycol | 3.00 |
| | Sodium Chloride | 4.00 |

The ingredients of Part A were mixed at room temperature. When homogeneous, the ingredients of Part B were added and pre-heated to 85 °C while mixing. The ingredients of Part C were added and pre-heated to 75 °C, slowly and gradually, while mixing with a mild turbine agitation, then a rapid turbine agitation. The whole mixture was cooled to 40°C. The water loss was compensated and stirring was continued while cooling to ambient temperature. Then the preparation was passed three times through an homogenizer.

### Example 11

A oil in water emulsion was prepared with the following ingredients

| Part | Ingredients | % w/w |
|---|---|---|
| A) | Compound of Example 1 | 5.00 |
| | PARSOL 340 | 5.00 |
| | PARSOL 1789 | 2.00 |
| | ARLACEL 481 | 9.00 |
| | ELFACOS C26 | 5.00 |
| | Petroleum Jelly | 2.00 |
| | Vaselin Oil | 10.00 |
| | CETIOL LC | 10.00 |
| | PHENONIP | 0.60 |
| | | |
| B) | Deionized Water | qsp 100 |
| | Sorbitol 70% | 5.00 |
| | Glycerin | 3.00 |
| | EDETA BD | 0.10 |

The organic phase A) containing the UV-filters was heated to 75°C, then the preheated aqueous phase B) (75°C) was added while stirring. The resulting emulsion was cooled to ambient temperature.

### Example 12

A lipstick was prepared with the following ingredients

| Ingredients | % w/w |
|---|---|
| Compound of Example 1 | 2.00 |
| PARSOL MCX | 2.00 |
| Satol | 20.00 |
| Ricinol | 12.00 |
| CUTINA HR | 3.00 |
| Texwax MH 181 | 30.00 |
| Vaselin Oil | qsp 100 |
| Petroleum Jelly | 11.00 |
| SILICONE 200/100 cs | 3.50 |
| Propylene Glycol | 3.00 |
| Butylated Hydroxytoluene | 0.02 |
| EDETA BD | 0.02 |

The ingredients were heated to 85°C while stirring, until complete dissolution was reached. When homogeneous the hot preparation was poured into mould.

## Claims

1. Sunscreen compositions containing in a cosmetically acceptable carrier
a) a compound of the general formula I 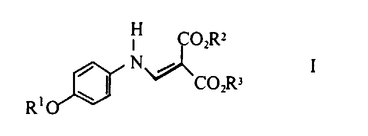 wherein
R¹ represents a C₁₋₈ straight or branched alkyl chain, and
R² and R³ each independently represent a C₁₋₈ straight or branched alkyl chain, and
b) common UV-B screening agents and/or common UV-A screening agents.

2. Sunscreen compositions according to claim 1 containing a compound of the general formula I, wherein R¹, R² and R³ each independently represent a straight C₁₋₄ alkyl chain, preferably ethyl.

3. Sunscreen compositions according to claim 1 or 2 containing a compound of the general formula I and a common UV-B screening agent.

4. Sunscreen compositions according to claim 3, wherein the UV-B screening agent is octylmethoxycinnamate.

5. Sunscreen compositions according to claim 3, wherein the UV-B screening agent is an organosiloxane of the general formula II 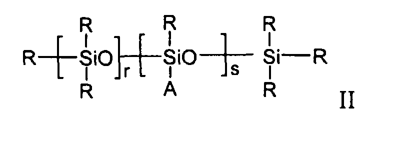 wherein
R signifies methyl;
A signifies a group of the formula IIa or IIb; 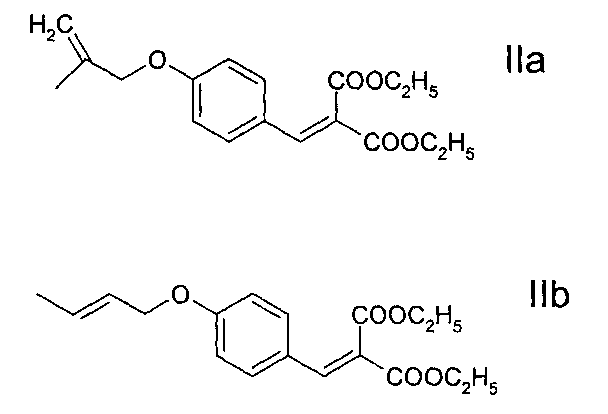
r is a statistical mean value of about 4;
s is a statistical mean value of about 60.

6. Sunscreen compositions according to claim 3, wherein the UV-B screening agent is microparticulated TiO₂.

7. Sunscreen compositions according to claim 3, wherein the UV-B screening agent is 4-methyl benzylidene camphor.

8. Sunscreen compositions according to claim 3, wherein the UV-B screening agent is octocrylene.

9. Sunscreen compositions according to claim 1 or 2 containing a compound of the general formula I and a common UV-A screening agent.

10. Sunscreen compositions according to claim 9, wherein the UV-A screening agent is 4-tert. butyl-4 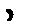 -methoxydibenzoyl-methane optionally stabilized by octocrylene, methylbenzylidene camphor or an organosiloxane of the general formula II.

11. Sunscreen compositions according to claim 9, wherein the UV-A screening agent is 2,2'-methylene-bis-(6-(2H-benzotriazole-2-yl)-4-(1,1,3,3,-tetramethylbutyl)-phenol.

12. Sunscreen compositions according to claim 9, wherein the UV-A screening agent is microparticulated ZnO.

13. Sunscreen compositions according to any one of claims 3 to 8 containing in addition a common UV-A screening agent, preferably 4-tert. butyl-4 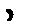 -methoxydibenzoyl-methane, 2,2'-methylene-bis-(6-(2H-benzotriazole-2-yl)-4-(1,1,3,3,-tetramethylbutyl)-phenol or microparticulated ZnO, most preferably 4-tert. butyl-4 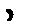 -methoxydibenzoylmethane.

14. Sunscreen compositions according to any one of claims 9 to 12 containing in addition a common UV-B screening agent, preferably is octylmethoxycinnamate, an organosiloxane of the general formula II or TiO₂.

15. Use of the sunscreen compositions according to anyone of claims 1 to 14 for the cosmetic treatment of human skin or hair when exposed to sun radiation.
